(19) Europäisches Patentamt
European Patent Office
Office européen des brevets

(11) **EP 2 419 074 B1**

(12) **EUROPÄISCHE PATENTSCHRIFT**

(45) Veröffentlichungstag und Bekanntmachung des Hinweises auf die Patenterteilung:
**03.05.2017 Patentblatt 2017/18**

(21) Anmeldenummer: **10710279.0**

(22) Anmeldetag: **17.03.2010**

(51) Int Cl.:
*A61K 8/89* *(2006.01)*     *A61Q 1/02* *(2006.01)*
*A61Q 5/02* *(2006.01)*     *A61Q 17/04* *(2006.01)*
*A61Q 19/04* *(2006.01)*    *A61Q 19/08* *(2006.01)*
*A61Q 9/02* *(2006.01)*     *A61Q 19/00* *(2006.01)*
*A61Q 19/10* *(2006.01)*    *B01F 17/54* *(2006.01)*
*C08G 77/04* *(2006.01)*    *C08G 77/00* *(2006.01)*
*A61K 8/892* *(2006.01)*    *A61K 8/894* *(2006.01)*
*A61Q 5/12* *(2006.01)*

(86) Internationale Anmeldenummer:
**PCT/EP2010/053422**

(87) Internationale Veröffentlichungsnummer:
**WO 2010/118926 (21.10.2010 Gazette 2010/42)**

(54) **VERWENDUNG ORGANOMODIFIZIERTER, IM SILICONTEIL VERZWEIGTER SILOXANE ZUR HERSTELLUNG KOSMETISCHER ODER PHARMAZEUTISCHER ZUSAMMENSETZUNGEN**

USE OF ORGANO-MODIFIED BRANCHED SILOXANES FOR THE PREPARATION OF COSMETIC OR PHARMACEUTICAL COMPOSITIONS

UTILISATION DES SILOXANES RAMIFIÉS ET ORGANO-MODIFIÉS POUR LA PREPARATION DES COMPOSITIONS COSMETIQUES OU PHARMACEUTIQUES

(84) Benannte Vertragsstaaten:
**AT BE BG CH CY CZ DE DK EE ES FI FR GB GR HR HU IE IS IT LI LT LU LV MC MK MT NL NO PL PT RO SE SI SK SM TR**

(30) Priorität: **16.04.2009 DE 102009002417**

(43) Veröffentlichungstag der Anmeldung:
**22.02.2012 Patentblatt 2012/08**

(73) Patentinhaber: **Evonik Degussa GmbH**
**45128 Essen (DE)**

(72) Erfinder:
• **HENNING, Frauke**
  **45130 Essen (DE)**
• **MEYER, Jürgen**
  **45134 Essen (DE)**
• **HARTUNG, Christian**
  **45133 Essen (DE)**
• **FERENZ, Michael**
  **45147 Essen (DE)**
• **KNOTT, Wilfried**
  **45355 Essen (DE)**
• **HERRWERTH, Sascha**
  **45134 Essen (DE)**

(56) Entgegenhaltungen:
**EP-A1- 2 159 248        EP-A1- 2 243 799**
**EP-A2- 1 679 335        EP-A2- 2 014 701**
**WO-A1-2009/065644       WO-A1-2009/138306**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

**Beschreibung**

Gebiet der Erfindung:

Die Erfindung betrifft die Verwendung organomodifizierter, im Siliconteil verzweigter Siloxane zur Herstellung kosmetischer, dermatologischer oder pharmazeutischer Zusammensetzungen. Insbesondere werden die Siloxane als Emulgatoren für W/O- und O/W-Emulsionen sowie als hydrophile und lipophile Pflegewirkstoffe zur Pflege von menschlichen oder tierischen Körperteilen eingesetzt.

Stand der Technik:

Organomodifizierte Siloxane werden in den verschiedensten Applikationen eingesetzt. Ihre Eigenschaften lassen sich unter anderem durch die Art der Modifikation, sowie durch die Modifikationsdichte gezielt einstellen.

So können zum Beispiel mit Allylpolyethern organophile oder nichtionische hydrophile Gruppen an ein Siloxangerüst gebunden werden. Derartige Verbindungen finden ihren Einsatz zum Beispiel als Polyurethan-Schaum-Stabilisatoren, als Entschäumer in Treibstoffen oder als Additive in Farben und Lacken.

Generell können Siloxane auch durch Umsetzung mit z.B. $\alpha$-Olefinen mit lipophilen Gruppen verknüpft werden. Die so erhaltenen Siliconwachse dienen zum Beispiel als Additiv in Personal-Care-Applikationen.

Es zeigt sich in vielen Anwendungsgebieten, dass die Wirkung des Siloxans entscheidend von der Verträglichkeit mit der entsprechenden Formulierung abhängt.

Als kosmetische Emulgatoren eignen sich zum Beispiel Siloxane, die neben aliphatischen, lipophilen Gruppen auf Basis von $\alpha$-Olefinen auch hydrophile Polyether tragen. Als typisches Beispiel ist hier das Verkaufsprodukt ABIL[®] EM 90 der Evonik Goldschmidt GmbH (Deutschland) zu nennen, das sich insbesondere durch eine hervorragende Stabilisierung von Wasser-in-Öl (W/O) Emulsionen auszeichnet.

Siloxanbasierte Emulgatoren für Öl-in-Wasser (O/W) Emulsionen müssen einen hydrophileren Charakter aufweisen, weshalb es sich bei diesen Produkten in der Regel um reine Polyethersiloxane handelt.

EP 1125574 beschreibt die Verwendung relativ hydrophober Polyethersiloxane als O/W-Emulgatoren, bei denen sich die Polyethergruppen $\alpha,\omega$- bzw. endständig am Siloxanrücken befinden. Diese Strukturen zeichnen sich durch eine lange ungestörte Siliconkette und damit insbesondere durch ein samtig seidiges Hautgefühl aus, das sie in kosmetische Emulsionen einzubringen vermögen.

Nachteilig an der Verwendung dieser Strukturen ist die oft nicht ausreichende Emulgierfähigkeit. Diese nicht ausreichende Emulgierfähigkeit läßt sich auf den geringen Substitutionsgrad zurückführen, da bei derartigen linearen Strukturen nur zwei Modifikationen an eine Siloxankette geknüpft werden können.

Um den Modifikationsgrad pro Siloxankette zu erhöhen, dabei aber den Siliconcharakter der Moleküle zu erhalten, wurden im Stand der Technik (z.B. EP 0298402) verzweigte Strukturen mit Hilfe von Vernetzungs(Cross-Linking)-Techniken hergestellt. Die Anzahl der möglichen Verzweigungseinheiten ist dabei begrenzt, da bei deren Synthese keine großen Mengen des Vernetzers (Cross-Linkers) eingesetzt werden können, ohne vergelungsfreie Produkte zu erhalten. Damit ist synthesebedingt die gezielte Einstellung hoher Verzweigungsgrade nicht möglich.

Eine weitere Möglichkeit, Verzweigungen in die Siloxanstruktur einzuführen, wird in EP 1416016 beschrieben. Statt der üblichen Vernetzer wie Divinylsiloxane oder Diolefine werden hier mono-vinyl-funktionelle Siloxane verwendet, um eine verzweigte Siliconstruktur zu generieren. Dieses Verfahren hat jedoch den Nachteil, dass mono-vinyl-funktionelle Siloxane aufwendig herzustellen und damit schwer zugänglich sind. Endständig funktionalisierte, im Siliconteil verzweigte Siloxane sind nach diesem Verfahren nicht zugänglich.

**Eine Aufgabe** der vorliegenden Erfindung bestand daher darin, neuartige, organomodifizierte, im Siliconteil verzweigte Siloxane zu entwickeln, die als leistungsfähige Additive für kosmetische und pharmazeutische Formulierungen eingesetzt werden können. Dies betrifft z.B. Emulgatoren, die sowohl in O/W als auch in W/O Emulsionen eingesetzt werden können, und dabei zudem in der Lage sind, ein samtig-seidiges Hautgefühl mit herausragenden Emulgiereigenschaften zu kombinieren.

US 5474712 beschreibt die Verwendung von Polyethersiloxanen nach dem Stand der Technik in Konditioniershampoos für Tiere.

WO 02/053111 beschreibt die Verwendung von Siliconpolyether-Block-Copolymeren mit (AB)$_n$ Strukturen in wässrigen, tensidischen Körperreinigungsmitteln, die gute kosmetische Eigenschaften speziell für das Volumen, die Kämmbarkeit und den Glanz von Haaren aufweisen.

US 5879671 beschreibt die Verwendung von wässrigen, tensidischen Körperreinigungsmitteln, die Mischungen von Amino-funktionellen Siloxanen und Polyethersiloxanen nach dem Stand der Technik als Pflegewirkstoff enthalten. Diese Mischungen bewirken eine dauerhafte Verbesserung der Trocken- und Naßkämmbarkeit des Haares.

DE 19603357 beschreibt die Verwendung von Polyethermodifizierten MQ-Harzen (Polyetherdimethylsiloxysilicat) in kosmetischen Formulierungen als Pflegewirkstoffe. Diese bewirken eine Förderung des Glanzes und der Beibehaltung von Locken.

Als Pflegewirkstoff für Haare und Haut sind im Artikel "Les Copolymeres Polysiloxanes polyethers comme additifs dans les formulations cosmetiques" (Dr. Kollmeier; Parfums, cosmetiques, aromes; 51; 1983; 67-72) Siloxane, die Polyethergruppen mittel- und/oder endständig tragen, beschrieben.

Kommerziell erhältliche Pflegewirkstoffe auf Siloxan-Basis sind zum Beispiel ABIL® B 8842, ABIL® B 88183 und ABIL® B 8832 (Evonik Goldschmidt GmbH), Belsil® DMC 6031, Belsil® DMC 6032 und Belsil® DMC 6033 (Wacker-Chemie). Das Verkaufsprodukt ABIL® B 8832 (Evonik Goldschmidt GmbH) ist ein endständig mit Polyethergruppen modifiziertes Siloxan, das als Pflegewirkstoff für Haare und Haut in tensidischen Lösungen und pflegenden kosmetischen Formulierungen eingesetzt wird. Die endständig mit Polyethergruppen modifizierten Siloxane sind eine leistungsfähige Klasse von Pflegewirkstoffen, die einen ausgeprägten konditionierenden Effekt für Haut und Haar aus kosmetischen Formulierungen bewirken. Ferner haben diese endständigen Siloxane Schaum verbessernde Eigenschaften. Jedoch sind, wie bereits weiter oben beschrieben, Siloxane mit sehr langem ungestörten Siloxanrückgrat (für ein gutes Hautgefühl) und mit mehr als zwei organischen Modifikationen (für die Kompatibilität in Formulierungen) in diesen klassischen Strukturen nicht zugänglich.

**Eine weitere Aufgabe** bestand daher darin, Verbindungen zu entwickeln, die als hydrophile oder lipophile Pflegewirkstoffe die sensorischen Eigenschaften kosmetischer und pharmazeutischer Formulierungen verbessern können.

Darüber hinaus sollten diese Siloxane vorzugsweise gute Kompatibilität mit der Formulierung aufweisen, einfach zu verarbeiten sein (flüssig bei Raumtemperatur) und mit herkömmlichen Siloxanen in Formulierungen kombiniert werden können.

Überraschenderweise wurde gefunden, dass organomodifizierte, im Siloxanteil verzweigte Siloxane, herstellbar nach dem in den Patentanmeldungen DE 102008041601.0 und DE 102007055485.2 beschriebenen Verfahren, sich als Additiv in kosmetischen, dermatologischen und pharmazeutischen Formulierungen eignen. Insbesondere eignen sie sich als leistungsfähige Additive, Emulgatoren und/oder Dispergierhilfsmittel. Des Weiteren eignen sie sich als Pflegewirkstoffe zur Verbesserung sensorischer Eigenschaften von kosmetischen, dermatologischen und pharmazeutischen Formulierungen.

Gegenstand der Erfindung ist die Verwendung von organomodifizierten, im Siloxanteil verzweigten Siloxanen gemäß Formel I in kosmetischen, dermatologischen und pharmazeutischen Formulierungen, wobei die Siloxane als Additive für kosmetische und pharmazeutische Formulierungen zur Verbesserung der sensorischen Eigenschaften und/oder zur Konditionierung von Haut und/oder Haar eingesetzt werden.

Ein weiterer Gegenstand sind Formulierungen enthaltend organomodifizierte, im Siliconteil verzweigte Siloxane der Formel I selbst sowie deren Verwendung

$$M_a \, M'_b \, D_c \, D'_d \, T_e \, Q_f \qquad \text{Formel I,}$$

wobei

$M = (R^1_3 \, Si \, O_{1/2})$

$M' = (R^2 R^1_2 \, Si \, O_{1/2})$

$D = (R^1_2 \, Si \, O_{2/2})$

$D' = (R^2 R^1 \, Si \, O_{2/2})$

$T = (R^3 \, Si \, O_{3/2})$

$Q = (Si \, O_{4/2})$

$a = 0$ bis 14;

$b = 1$ bis 14;

mit der Maßgabe, dass

$$a + b > 3;$$

$c = 20$ bis 400;

$d = 0$

$e = 0$ bis 15;

$f = 0$ bis 10;

mit der Maßgabe, dass

$$e + f \geq 3;$$

$R^1$ = Methyl;

$R^2$ = unabhängig voneinander gleiche oder verschiedene lineare oder verzweigte Alkylreste mit mehr als 7 Kohlenstoffatomen oder Polyetherreste der allgemeinen Formel IIa,

$$-CH_2-CH_2-(CH_2)_nO(EO)_x(PO)_y(XO)_zR^4 \qquad \text{Formel IIa,}$$

mit

$EO$ = $(C_2H_4O)$ ;

$PO$ = $(C_3H_6O)$;

$XO$ = $(C_2H_3R^5O)$ ;

$n$ = 0 bis 9, insbesondere 1;

$x$ = 5 bis 30;

$y$ = 0;

$z$ = 0;

$R^4$ = H;

$R^5$ = unabhängig voneinander gleiche oder verschiedene Reste ausgewählt aus der Gruppe umfassend: Alkylreste mit 2 bis 16 C-Atomen, die durch Etherfunktionen unterbrochen sein können, Alkarylreste mit 7 bis 18 C-Atomen, Arylreste mit 6 bis 16 C-Atomen, bevorzugt Ethyl oder Phenyl;

$R^3$ = unabhängig voneinander gleiche oder verschiedene Reste $R^1$ oder $R^2$, bevorzugt $R^1$, insbesondere Methyl, Dodecyl oder Hexadecyl

sind.

Ein weiterer Gegenstand der Erfindung ist die Verwendung von organomodifizierten, im Siliconteil verzweigten Siloxanen in kosmetischen, dermatologischen und pharmazeutischen Formulierungen, dadurch gekennzeichnet, dass diese Siloxane hergestellt werden durch Hydrosilylierung von ungesättigten Polyetherderivaten mit im Siliconteil verzweigten, bei einer Temperatur von 25 °C und einem Druck von 101325 Pa flüssigen SiH-funktionellen Siloxanblockcopolymeren, wobei die Siloxanblockcopolymere hergestellt wurden, indem eine Mischung, enthaltend

    a) ein oder mehrere SiH-funktionelle Siloxane,
    b) ein oder mehrere SiH-Funktion-freie Siloxane und
    c) ein oder mehrere Tetraalkoxysilane, und gegebenenfalls
    d) ein oder mehrere Trialkoxysilane

unter Zugabe von Wasser und in Anwesenheit von mindestens einem festen Brönstedt-sauren Katalysator, welcher ausgewählt ist aus den sauren Ionenaustauschern, in einem Verfahrensschritt umgesetzt werden.

Dieses Herstellungsverfahren der SiH-funktionellen Siloxanblockcopolymere ist in den Patentanmeldungen DE 102008041601.0 und DE 102007055485.2 beschrieben.

Gesteuert über die Reaktionsbedingungen, wie beispielsweise die Wassermenge oder die Reaktionstemperatur, können die im Silikonteil verzweigten Siloxane noch Restanteile an Alkoxygruppen aufweisen.

Die erfindungsgemäßen Strukturen besitzen trotz hohem Verzweigungsgrad und teils hohen Molekulargewichten eine vergleichsweise niedrige Viskosität, so dass sie gut verarbeitbar sind. Die Viskositäten dieser Verbindungen liegen unterhalb von 25.000 mPas.

Erfindungsgemäße Siloxan-Zubereitungen oder Zusammensetzungen eignen sich u.a. als Emulgatoren, die hervorragende emulgierende und emulsionsstabilisierende Eigenschaften aufweisen. Um als Emulgator geeignet zu sein, muß durch die Organomodifizierung eine bestimmte Balance zwischen Hydrophobie und -philie eingestellt werden. Ein weiterer Gegenstand ist daher die Verwendung der oben beschriebenen Siloxane als Emulgatoren, insbesondere in Öl-in-Wasser- (O/W) und/oder Wasser-in-Öl- (W/O) Emulsionen. Insbesondere können die Formulierungen anorganische oder organische Pigmente und/oder anorganische oder organische Partikel enthalten sein.

Zudem eignen sich die Siloxane in den erfindungsgemäßen Zubereitungen oder Zusammensetzungen als Additive für kosmetische und pharmazeutische Formulierungen zur Verbesserung ihrer sensorischen Eigenschaften. Auch die als Emulgator eingesetzten Systeme ermöglichen es, in kosmetischen Formulierungen ein samtig-seidiges Hautgefühl einzubringen. Dies ist insofern wichtig, da Konsumenten zunehmend Wert darauf legen, dass kosmetische Formulierungen sich nicht nur leicht verteilen lassen und gut in die Haut einziehen, sondern dass nach dem Einziehen ein glatter, weicher, samtiger Eindruck verbleibt. Der Einsatz der erfindungsgemäße Siloxane als Additive für kosmetische und pharmazeutische Formulierungen zur Verbesserung ihrer sensorischen Eigenschaften ist daher ein weiterer Gegenstand dieser Erfindung.

Vorteile der erfindungsgemäßen Verwendung sind, dass die Siloxane als leistungsfähige Emulgatoren und als leistungsfähige Pflegewirkstoffe eingesetzt werden können. Dabei bewirken sie einen ausgeprägten konditionierenden Effekt für Haut und Haar. Durch diesen konditionierenden Effekt auf beispielsweise der Haut kann einem trockenen, spröden oder rauen Zustand der Haut nach Anwendungen der Formulierung vorgebeugt werden und ein angenehmes, samtig-seidiges Hautgefühl erzielt wird.

Ein weiterer Vorteil ist, dass die erfindungsgemäße Verwendung als Pflegewirkstoff zu einem verbesserten Anschäumverhalten, einem erhöhten Schaumvolumen und einer besseren Schaumcremigkeit der Formulierungen beiträgt.

Ein weiterer Vorteil ist, dass die verwendeten Siloxane einfach zu verarbeiten sind, da sie bei Raumtemperatur flüssig oder leicht zu verflüssigen sind und mit herkömmlichen Bestandteilen von kosmetischen und pharmazeutischen Formulierungen kombiniert werden können.

Das in DE 102008041601.0 und DE 102007055485.2 beschriebene Herstellungsverfahren hat hinsichtlich der Qualität und Lagerstabilität der Endprodukte den Vorteil, dass die erfindungsgemäß hergestellten verzweigten Wasserstoffsiloxane und die daraus gefertigten Folgeprodukte keine bzw. kaum Vergelungsneigung besitzen und somit über einen längeren Zeitraum gelagert werden können, ohne dass sich die Viskosität der Produkte maßgeblich verändert. Dieser Vorteil ist speziell bei hochmolekularen Produkten hervorzuheben. Die erfindungsgemäßen Produkte basieren somit vor allem auf organomodifizierten, im Siliconteil verzweigten Siloxanen und damit hochverzweigten und auch höhermolekulareren (durchschnittliche Molmasse >3000 g/mol), dabei jedoch vergelungsfreien und damit vergleichsweise niedrigviskosen (dynamische Viskosität <25000 mPas) Strukturen.

Ein weiterer Vorteil der erfindungsgemäßen Siloxane ist, dass die Vorteile von seitenständig modifizierten Siloxanen und von $\alpha,\omega$-modifizierten Siloxanen in ihnen vereint vorliegen und ein höherer Modifikationsgrad im Sinne einer größeren Anzahl an Substitutionsmöglichkeiten gegeben ist im Vergleich zu rein linearen Strukturen. Hierdurch sind Strukturen mit langem ungestörten Siloxanrückgrat zugänglich, die ein besonderes gutes Hautgefühl in kosmetischen, dermatologischen und pharmazeutischen Formulierungen einzubringen vermögen. Die Formulierungen zeichnen sich durch ein samtig-seidiges sowie wenig öliges Hautgefühl aus.

Die zur Organomodifizierung der Siloxane verwendeten ungesättigten organischen Verbindungen, die zumindest eine Doppelbindung pro Molekül aufweisen, können z.B. $\alpha$-Olefine, Vinylpolyoxyalkylene, Allylpolyoxyalkylene, Glycerinmonoallylether und/oder Polyglycerinmonoallylether sein. Vorzugsweise werden Vinylpolyoxyalkylene und/oder Allylpolyoxyalkylene eingesetzt. Besonders bevorzugte Vinylpolyoxyalkylene sind z.B. Vinylpolyoxyalkylene mit einem Molgewicht im Bereich von 100 g/mol bis 5.000 g/mol, die aus den Monomeren Propylenoxid, Ethylenoxid, Butylenoxid und/oder Styroloxid blockweise oder statistisch verteilt aufgebaut sein können und die sowohl hydroxyfunktionell als auch durch eine Methyletherfunktion oder eine Acetoxyfunktion endverkappt sein können. Besonders bevorzugte Allylpolyoxyalkylene sind z.B. Allylpolyoxyalkylene mit einem Molgewicht im Bereich von 100 g/mol bis 5.000 g/mol, die aus den Monomeren Propylenoxid, Ethylenoxid, Butylenoxid und/oder Styroloxid blockweise oder statistisch verteilt aufgebaut sein können und die sowohl hydroxyfunktionell als auch durch eine Methyletherfunktion oder eine Acetoxyfunktion endverkappt sein können. Besonders bevorzugt können weiterhin Glycerinmonoallylether und Polyglycerinmonoallylether eingesetzt werden.

Die edelmetallkatalysierte Hydrosilylierung der verzweigten Wasserstoffsiloxane mit diesen ungesättigten organischen Verbindungen kann z.B. wie im Stand der Technik, z.B. in EP 1520870, beschrieben durchgeführt werden.

Die platinkatalysierte Hydrosilylierung weist eine hohe Selektivität bei der Bildung der gewünschten SiC-Bindungen auf. In Abhängigkeit von den Reaktionsbedingungen können jedoch Nebenreaktionen, wie beispielsweise eine dehydrogenative SiOC-Verknüpfung der SiH-Gruppen mit Hydroxygruppen oder auch eine Hydrolyse- und Kondensationsreaktion zur Bildung von Nebenprodukten führen.

Es versteht sich von selbst, dass durch das Abmischen der organo- und insbesondere polyethermodifizierten, verzweigten Siloxane mit unverzweigten Siloxanen Gemische erhalten werden können, die wertvolle grenzflächenaktive Wirkstoffe sind, die aber, je nach Mischungsverhältnis, in der Summe weniger als eine Verzweigungseinheit pro Molekül aufweisen können.

**Anwendung:**

Die Verwendung der erfindungsgemäßen Zubereitungen wird nachfolgend beispielhaft beschrieben, ohne dass die Erfindung auf diese beispielhaften Ausführungsformen beschränkt sein soll. Sind nachfolgend Bereiche, allgemeine Formeln oder Verbindungsklassen angegeben, so sollen diese nicht nur die entsprechenden Bereiche oder Gruppen von Verbindungen umfassen, die explizit erwähnt sind, sondern auch alle Teilbereiche und Teilgruppen von Verbindungen, die durch Herausnahme von einzelnen Werten (Bereichen) oder Verbindungen erhalten werden können.

Alle Prozentangaben in der nachfolgenden Beschreibung oder den Beispielen sind als auf das Gewicht bezogen zu verstehen, soweit nichts anderes explizit angegeben ist.

**Anwendung als Emulgatoren:**

Die erfindungsgemäßen Emulgator-Formulierungen werden vorzugsweise als Öl-in-Wasser-, Wasser-in-Öl- oder Wasser-in-Silicon-Emulgatoren oder Dispergierhilfsmittel verwendet. Gegenstand der vorliegenden Erfindung sind deshalb auch anorganische oder organische Pigmente oder Partikel enthaltende Dispersionen oder Emulsionen, enthaltend mindestens einen der erfindungsgemäßen Emulgatoren.

Bevorzugt wird der Emulgator auch zur Herstellung von O/W-Tränkemulsionen für Textilien verwendet. Bevorzugt handelt es sich bei den Textilien um Feuchttücher, besonders bevorzugt um kosmetische Feuchttücher.

Die erfindungsgemäßen Emulsionen und Dispersionen enthalten bezogen auf die Gesamtmasse mehr Massenprozent Ölkomponente als die Summe der Massenprozente von Emulgator, Tensid und ggfs. Co-Emulgator.

Ein weiterer Gegenstand der Erfindung ist die Verwendung der erfindungsgemäßen Emulgator-Formulierungen zur Herstellung kosmetischer, dermatologischer oder pharmazeutischer Formulierungen. Ebenso ist Gegenstand der Erfindung die Verwendung der erfindungsgemäßen Emulsionen und Dispersionen zur Herstellung kosmetischer, dermatologischer oder pharmazeutischer Formulierungen.

Somit ist die kosmetische, dermatologische oder pharmazeutische Formulierung enthaltend mindestens eine erfindungsgemäße Emulgator-Formulierung oder mindestens eine erfindungsgemäße Emulsion oder Dispersion ebenfalls Gegenstand der Erfindung.

Die erfindungsgemäßen kosmetischen, dermatologischen oder pharmazeutischen Formulierungen sowie die Pflege- und Reinigungsmittel können z.B. mindestens eine zusätzliche Komponente enthalten, ausgewählt aus der Gruppe der Emollients, Emulgatoren und Tenside, Verdicker/ Viskositätsregler/Stabilisatoren, UV-Lichtschutzfilter, Antioxidantien, Hydrotrope (oder Polyole), Fest- und Füllstoffe, Filmbildner, Perlglanzadditive, Deodorant- und Antitranspirantwirkstoffe, Insektrepellentien, Selbstbräuner, Konservierungsstoffe, Konditioniermittel, Parfüme, Farbstoffe, kosmetische Wirkstoffe, Pflegeadditive, Überfettungsmittel, Lösungsmittel.

Substanzen, die als beispielhafte Vertreter der einzelnen Gruppen eingesetzt werden können, sind dem Fachmann bekannt und können beispielsweise der deutschen Anmeldung DE 102008001788.4 entnommen werden.

In einer bevorzugten Ausführungsform enthalten die erfindungsgemäßen kosmetischen, dermatologischen oder pharmazeutischen Formulierungen als zusätzliche Komponente Pigmente (z.B. $TiO_2$, $FeO_x$, ZnO, Mica und beispielsweise solche, die unter UV-Filtersubstanzen und Feststoffe gelistet sind) oder Partikel (z.B. Siliconelastomere, Nylon-12, PMMA, Bornitrid und beispielsweise solche, die unter UV-Filtersubstanzen und Feststoffe gelistet sind).

In einer ebenso bevorzugten Ausführungsform enthalten die erfindungsgemäßen kosmetischen, dermatologischen oder pharmazeutischen Formulierungen als zusätzliche Komponente kosmetische Wirkstoffe.

Bevorzugt ist die Verwendung erfindungsgemäßer Emulgator-Formulierungen oder der erfindungsgemäßen Emulsion oder Dispersion zur Herstellung kosmetischer oder pharmazeutischer Formulierungen.

Die erfindungsgemäßen Emulgator-Formulierungen und Zubereitungen können in Abhängigkeit ihrer Hydrophilie sowohl in der Form von W/O- als auch in der Form von O/W-Emulgator-Formulierungen eingesetzt werden.

Als Applikationsformen der Emulsionen und Dispersionen enthaltend die erfindungsgemäßen Emulgator-Formulierungen sind daher Sprays, Lotionen, Cremes, Salben und somit der Einsatz über einen sehr breiten Konsistenzbereich von wasserdünn bis stark pastös, im Extremfall sogar fest, möglich.

Daher können die Emulgator-Formulierungen und Zubereitungen beispielsweise in Pflegecremes und -lotionen für Gesicht, Körper und Hände, in Sonnenschutzemulsionen, in Schminken, in Aersolen, Roll-ons, Pumpsprays, Stiften z.B. im AP/Deo-Bereich, in Babypflegeprodukten, in Intimpflege-, Fußpflege-, Haarpflege-, Nagelpflege-, Zahnpflege- oder Mundpflegeprodukten sowie in dermatologischen Salben eingesetzt werden.

**Anwendung als lipophile Pflegewirkstoffe:**

Die erfindungsgemäßen Siliconprodukte werden zudem vorzugsweise als lipophile Emollients verwendet. Gegenstand der vorliegenden Erfindung sind deshalb auch kosmetische Formulierungen, in denen die erfindungsgemäßen Siliconprodukte als Additiv zur Verbesserung der sensorischen Eigenschaften eingesetzt werden. Die hydrophoben Emollients/Pflegewirkstoffe sind vorzugsweise in der Ölphase der kosmetischen Formulierung gelöst. Verwendung finden hier v.a. Siliconprodukte, die mit lipophilen Substituenten wie z.B. Alkylgruppen modifiziert sind.

**Anwendung als hydrophile Pflegewirkstoffe:**

Ein weiterer Bestandteil der Erfindung sind Pflegeformulierungen enthaltend die erfindungsgemäßen Pflegewirkstoffe.

Die erfindungsgemäßen Siliconprodukte werden u.a. auch vorzugsweise als hydrophile Emollients verwendet. Gegenstand der vorliegenden Erfindung sind deshalb auch kosmetische Formulierungen, in denen die erfindungsgemäßen Siliconprodukte als Additiv zur Verbesserung der sensorischen Eigenschaften eingesetzt werden. Für diese Anwendung ist eine ausreichende Wasserlöslichkeit oder Wasseremulgierbarkeit der Siliconprodukte nötig, so dass v.a. mit Polyethergruppen modifizierte Produkte hier Einsatz finden.

Bevorzugt enthalten die Pflegeformulierungen von 0,01 Massenprozent bis 20 Massenprozent, bevorzugt 0,05 Massenprozent bis 10 Massenprozent, besonders bevorzugt 0,1 Massenprozent bis 3 Massenprozent Pflegewirkstoff bezogen auf die Gesamtmasse der Pflegeformulierung.

Ebenfalls bevorzugt sind wässrige, besonders bevorzugt wässrige, tensidische Pflegeformulierungen.

Bevorzugt sind diese Pflegeformulierungen kosmetische, dermatologische oder pharmazeutische Formulierungen.

Diese können beispielsweise sein: Duschbäder und -gele, Badeformulierungen, Flüssigseifen und Shampoos, Hautmasken, Rasierschäume, Haarspülungen, Leave-in Conditioner und Stylingprodukte für Haar, wobei unter dem Begriff Haar auch Fell und Pelz zu verstehen ist.

Die erfindungsgemäßen Pflegeformulierungen können wie oben beschrieben z.B. mindestens eine zusätzliche Komponente nach Anmeldung DE 102008001788.4 enthalten.

Erfindungsgemäße Pflegeformulierungen können Verwendung als Hautpflege-, Gesichtspflege-, Kopfpflege-, Körperpflege, Intimpflege-, Fußpflege-, Haarpflege-, Nagelpflege, Zahnpflege- oder Mundpflegeprodukt finden.

Erfindungsgemäße Pflegeformulierungen können Verwendung in Form einer Emulsion, einer Suspension, einer Lösung, einer Creme, einer Salbe, einer Paste, eines Gels, eines Aerosols, eines Sprays, eines Reinigungsproduktes, eines Schmink- oder Sonnenschutzpräparates oder eines Gesichtswassers finden.

Pflegeformulierungen entsprechend der vorliegenden Erfindung verfügen über einen konditionierenden Effekt auf Haut und Haaren, wobei unter dem Begriff Haar auch Fell oder Pelz als subsummiert zu verstehen ist.

Daher ist ein Gegenstand der Erfindung die Verwendung erfindungsgemäßer Verbindungen und deren Formulierungen zur Konditionierung von Haut und/oder Haaren; ein weiterer Gegenstand der Erfindung ist die Verwendung erfindungsgemäßer Formulierungen zur Vermittelung eines angenehmen, samtig seidigen Hautgefühls und zur Vermeidung eines trockenen oder spröden Zustands der Haut; noch ein weiterer Gegenstand der Erfindung ist die Verwendung erfindungsgemäßer Formulierungen zur Verbesserung mindestens einer Eigenschaft der Haare ausgewählt aus der Gruppe: Kämmbarkeit, Weichheit, Formbarkeit, Handhabbarkeit, Entwirrbarkeit, Volumen, Glanz.

Erfindungsgemäße Pflegeformulierungen erniedrigen die Rauhigkeit strapazierter Haut. Daher ist ein weiterer Gegenstand der Erfindung die Verwendung der erfindungsgemäßen Formulierungen zur Verringerung der Hautrauhigkeit.

Ein weiterer Gegenstand der Erfindung sind Zubereitungen enthaltend organomodifizierte, im Siliconteil verzweigte Siloxane der Formel I.

Ein weiterer Gegenstand der Erfindung sind kosmetische, dermatologische oder pharmazeutische Formulierungen enthaltend mindestens eine der Zubereitungen oder Formulierungen in Form einer Emulsion.

Ein weiterer Gegenstand der Erfindung sind kosmetische, dermatologische oder pharmazeutische Formulierungen oder Zubereitungen als zusätzliche Komponente Partikel oder Pigmente.

Ein weiterer Gegenstand der Erfindung sind kosmetische, dermatologische oder pharmazeutische Formulierungen enthaltend als zusätzliche Komponente kosmetische Wirkstoffe.

Beispiele:

Werden im Rahmen der vorliegenden Erfindung Verbindungen, wie z.B. organomodifizierte Polysiloxane oder Polyoxyalkylen, beschrieben, die verschiedene Monomereinheiten mehrfach aufweisen können, so können diese statistisch verteilt (statistisches Oligomer) oder geordnet (Blockoligomer) in diesen Verbindungen vorkommen. Angaben zu Anzahl von Einheiten in solchen Verbindungen sind als statistische Mittelwert, gemittelt über alle entsprechenden Verbindungen, zu verstehen. Als Emulgator-Formulierung ist im Rahmen dieser Erfindung ein Emulgator zu verstehen, der mindestens eine Substanz der allgemeinen Formel (I) und gegebenenfalls mindestens einen Co-Emulgator enthält.

**Allgemeine Arbeitsvorschrift I (AAV I) zur Herstellung polyether-, alkyl- bzw. glycerin-modifizierter Siloxane nach Formel I:**

$$M_a \, M'_b \, D_c \, D'_d \, T_e \, Q_f \qquad \text{Formel I,}$$

wobei

M = $(R^1_3 Si O_{1/2})$
M' = $(R^2R^1_2 Si O_{1/2})$
D = $(R^1_2 Si O_{2/2})$
D' = $(R^2R^1 Si O_{2/2})$
T = $(R^3 Si O_{3/2})$
Q = $(Si O_{4/2})$

mit

$R^1$ = Methyl,
$R^2$, $R^3$: siehe Tabelle 1

gilt.

In einem Vierhalskolben, versehen mit KPG-Rührer, Tropftrichter, Innenthermometer und Rückflußkühler wurden 1,3-(b+d) mol α-Olefin bzw. Allylpolyether bzw. Glycerinmonoallylether und 10 ppm Karstedt-Katalysator vorgelegt und unter Stickstoffatmosphäre auf 90 °C erhitzt. 1 mol des verzweigten SiH-Siloxans der allgemeinen Formel IV

$$[Me_3SiO_{1/2}]_a[Me_2HSiO_{1/2}]_b[Me_2SiO_{2/2}]_c[MeHSiO_{2/2}]_d[R^3SiO_{3/2}]_e[SiO_{4/2}]_f \qquad \textbf{Formel IV}$$

(hergestellt nach Vorschrift der Patentanmeldungen DE 102008041601.0 und DE 102007055485.2) wurde zugetropft und der Ansatz für 2 h bei 90 °C gerührt. Laut SiH-Wert-Bestimmung wurde ein vollständiger Umsatz des SiH-Siloxans erhalten. Es wurde jeweils ein viskoses, klar bis leicht trübes, fast farbloses Produkt erhalten.

In Tabelle 1 sind die nach dieser allgemeinen Vorschrift AAV I hergestellten Siloxane aufgeführt.

Tabelle 1:

| Hergestellte polyether-, alkyl- bzw. glycerin-modifizierte Siliconprodukte nach Formel I ($R^1$ = Methyl) | | | | | | | | | | |
|---|---|---|---|---|---|---|---|---|---|---|
| **Produkt Nr.** | **Modifizierung $R^2$** | | | $R^3$ | a | b | c | d | e | f |
| | **$-CH_2CH_2CH_2O(EO)_x(PO)_yR^4$** | | | | | | | | | |
| | x | y | $R^4$ | | | | | | | |
| 1 | 10 | 10 | H | $CH_3$ | 0 | 7 | 78 | 0 | 5 | 0 |
| 2 | 10 | 10 | H | $CH_3$ | 0 | 7 | 138 | 0 | 5 | 0 |
| 3 | 10 | 10 | H | $CH_3$ | 0 | 7 | 238 | 0 | 5 | 0 |
| 4 | 10 | 10 | H | $CH_3$ | 0 | 5 | 142 | 0 | 3 | 0 |
| 5 | 21 | 5 | H | $CH_3$ | 0 | 7 | 138 | 0 | 5 | 0 |
| 6 | 13 | 0 | H | $C_{16}H_{33}$ | 0 | 7 | 138 | 0 | 5 | 0 |
| 7 | 0 | 17 | $CH_3$ | $CH_3$ | 0 | 7 | 238 | 0 | 5 | 0 |
| 8 | 10 | 10 | H | $CH_3$ | 6 | 4 | 133 | 4 | 0 | 3 |
| | | | | | | | | | | |
| 9 | **$-CH_2CH_2CH_2OCH_2CH(OH)CH_2OH$** | | | $C_{16}H_{33}$ | 0 | 7 | 138 | 0 | 5 | 0 |
| 10 | **$-CH_2CH_2CH_2OCH_2CH(OH)CH_2OH$** | | | $CH_3$ | 0 | 7 | 238 | 0 | 5 | 0 |
| | | | | | | | | | | |
| 11 | $C_{16}H_{33}$ | | | $CH_3$ | 0 | 7 | 238 | 0 | 5 | 0 |
| | | | | | | | | | | |
| **Vergleichsbeispiel:** | **$-CH_2CH_2CH_2O(EO)_x(PO)_yR^4$** | | | | | | | | | |
| | x | y | $R^4$ | | | | | | | |
| 12 | 21 | 5 | H | $CH_3$ | 2 | 0 | 75 | 5 | 0 | 0 |

Nicht erfindungsgemäß sind die Beispiele der Tabelle 1: 7 bis 10 und 12, sowie der Tabelle 2: 15 bis 17.

Allgemeine Arbeitsvorschrift II (AAV II) zur Herstellung gemischte alkyl- und polyether-modifizierter bzw. gemischt alkyl- und glycerin-modifizierter Siloxane nach Formel I:

$$M_a\ M'_b\ D_c\ D'_d\ T_e\ Q_f \qquad \textbf{Formel I,}$$

wobei

$M$ $= (R^1{}_3\ Si\ O_{1/2})$
$M'$ $= (R^2R^1{}_2\ Si\ O_{1/2})$
$D$ $= (R^1{}_2\ Si\ O_{2/2})$
$D'$ $= (R^2R^1\ Si\ O_{2/2})$
$T$ $= (R^3\ Si\ O_{3/2})$
$Q$ $= (Si\ O_{4/2})$

mit

$R^1 = R^3 =$ Methyl,
$R^2:$ siehe Tabelle 2

gilt.

In einem Vierhalskolben, versehen mit KPG-Rührer, Tropftrichter, Innenthermometer und Rückflußkühler wurden 1,3·(b+d) ·o mol α-Olefin sowie 1,3·(b+d) ·p mol Allylpolyether bzw. Glycerinmonoallylether (mit o+p=1) und 10 ppm Karstedt-Katalysator (bezogen auf die Rohstoffe ohne Lösungsmittel) in ca. 30% Toluol (bezogen auf die Gesamteinwaage) vorgelegt und unter Stickstoffatmosphäre auf 90 °C erhitzt. 1 mol des verzweigten SiH-Siloxans der allgemeinen Formel V

$$[Me_3SiO_{1/2}]_a[Me_2HSiO_{1/2}]_b[Me_2SiO_{2/2}]_c[MeHSiO_{2/2}]_d[MeSiO_{3/2}]_e[SiO_{4/2}]_f \qquad \textbf{Formel V}$$

(hergestellt nach Vorschrift der Patentanmeldungen DE 102008041601.0 und DE 102007055485.2) wurde zugetropft und der Ansatz für 2 h bei 90 °C gerührt. Laut SiH-Wert-Bestimmung wurde ein vollständiger Umsatz des SiH-Siloxans erhalten.

Flüchtige Anteile wurden anschließend im Vakuum bei ca. 1 mbar und 110 °C abdestilliert. Es wurde jeweils ein viskoses, klar bis leicht trübes, fast farbloses Produkt erhalten.

In Tabelle 2 sind die nach dieser allgemeinen Vorschrift AAV II hergestellten Siloxane aufgeführt.

Tabelle 2:

| Hergestellte gemischt alkyl- und polyether-modifizierter bzw. gemischt alkyl- und glycerin-modifizierte Siliconprodukte nach Formel I ($R^1 = R^3$ = Methyl) | | | | | | | | | | | |
|---|---|---|---|---|---|---|---|---|---|---|---|
| **Produkt Nr.** | **Modifizierung $R^2$** | | | **p/o** | **a** | **b** | **c** | **d** | **e** | **f** |
| | $-CH_2CH_2CH_2O(EO)_x(PO)_yH$ | | **Alkyl** | | | | | | | |
| | **x** | **y** | | | | | | | | |
| 13 | 8 | 0 | $C_{16}H_{33}$ | 1/3 | 0 | 12 | 228 | 0 | 10 | 0 |
| 14 | 8 | 0 | $C_{16}H_{33}$ | 1/3 | 0 | 16 | 227 | 0 | 0 | 7 |
| 15 | 8 | 0 | $C_{16}H_{33}$ | 1/5 | 8 | 0 | 73 | 25 | 0 | 3 |
| | | | | | | | | | | |
| 16 | $-CH_2CH_2CH_2OCH_2CH(OH)CH_2OH$ | | $C_{12}H_{25}$ | 9/15 | 0 | 12 | 228 | 0 | 10 | 0 |
| 17 | $-CH_2CH_2CH_2OCH_2CH(OH)CH_2OH$ | | $C_{12}H_{25}$ | 9/16 | 8 | 0 | 73 | 25 | 0 | 3 |

Anwendungsbeispiele:

Alle Konzentrationen in den Anwendungsbeispielen sind in Gewichtsprozent angegeben. Zur Herstellung der Emulsionen wurden dem Fachmann bekannte übliche Homogenisierverfahren eingesetzt.

Die Formulierungsbestandteile sind in den Zusammensetzungen in Form der allgemein anerkannten INCI-Nomenklatur unter Verwendung der englischen Begriffe benannt.

Emulsionsbeispiele:

Diese Beispiele sollen zeigen, dass die erfindungsgemäßen Emulgatoren in einer Vielzahl kosmetischer Formulierungen eingesetzt werden können. Je nach Hydrophilie der erfindungsgemäßen Emulgatoren können O/W oder W/O Emulsionen hergestellt werden. Es ist darüber hinaus mit Hilfe der erfindungsgemäßen Emulgatoren möglich, Pigmente oder Festkörper stabil in Emulsionspräparate einzuarbeiten. Weiterhin zeigen die Beispiele die gute Kompatibilität mit typischen Coemulgatoren, Ölen, Verdickern und Stabilisatoren.

O/W Emulsionsbeispiele:

Tabelle 3: Tagescreme gegen Hautalterung (anti-Aging):

| Beispiel | **1** |
|---|---|
| Emulgator 1 | 1,50% |
| Ceteareth-25 | 1,00% |
| Stearyl Alcohol | 1,50% |
| Glyceryl Stearate | 3,00% |
| Stearic Acid | 1,50% |
| Myristyl Myristate | 1,00% |
| Ceramide IIIB | 0,10% |
| Caprylic/Capric Triglyceride | 5,00% |
| Ethylhexyl Palmitate | 4,40% |
| Ethylhexyl Methoxycinnamate | 2,00% |
| Butyl Methoxydibenzoyl-methane | 1,00% |
| Glycerin | 3,00% |
| Water | ad 100% |
| TEGO® Carbomer 134 (Carbomer) | 0,10% |
| Ethylhexyl Palmitate | 0,40% |
| Sodium Hydroxide (10% in water) | q.s. * |
| Preservative | q.s. |
| Perfume | q.s. |
| * q.s. = quantum satis (lat.: so viel wie nötig = ausreichende Menge) | |

Tabelle 4:

| Selbstbräunende Köperlotion: | |
|---|---|
| Beispiel | **2** |
| Emulgator 5 | 1,00% |
| ABIL® Care XL 80 (Bis-PEG/PPG-20/5 PEG/PPG-20/5 Dimethicone; Methoxy PEG/PPG-25/4 Dimethicone; Caprylic/Capric Triglyceride) | 1,00% |
| Cetearyl Isononanoate | 5,00% |

(fortgesetzt)

| Selbstbräunende Köperlotion: | |
|---|---|
| Beispiel | **2** |
| Decyl Cocoate | 5,00% |
| Isopropyl Myristate | 5,00% |
| Sepigel® 305 (Polyacrylamide; C13-14 Isoparaffin; Laureth-7) | 1,50% |
| PEG-30 Glyceryl Stearate | 2,00% |
| Dihydroxyacetone | 5,00% |
| Propylene Glycol | 3,00% |
| Water | ad 100% |
| Citric Acid | q.s. |
| Preservative | q.s. |
| Perfume | q.s. |

Tabelle 5:

| Kationische Sonnenschutzcreme (in-vitro SPF 18): | |
|---|---|
| Beispiel 3 | 3 |
| Emulgator 1 | 2,00% |
| Distearyldimonium Chloride | 1,50% |
| Glyceryl Stearate | 2,00% |
| Stearyl Alcohol | 1,00% |
| C12-15 Alkyl Benzoate | 5,00% |
| TEGO® Sun TDEC 45 (Tita-nium Dioxide; Diethylhexyl Carbonate; Poly-glyceryl-6 Polyhydroxystearate)) | 5,00% |
| Diethylhexyl Carbonate | 3,50% |
| Cetyl Ricinoleate | 1,00% |
| Triisostearin | 1,00% |
| Octocrylene | 3,00% |
| Ethylhexyl Methoxycinna-mate | 4,00% |
| Butyl Methoxydibenzoyl-methane | 2,00% |
| Water | ad 100% |
| Glycerin | 3,00% |
| Preservative | q.s. |
| Perfume | q.s. |

Tabelle 6:

| Hautglättende Körperlotion (Body Lotion): | |
|---|---|
| Beispiel | **4** |
| Emulgator 5 | 2,50% |

(fortgesetzt)

| Hautglättende Körperlotion (Body Lotion): | |
|---|---|
| Beispiel | **4** |
| Diethylhexyl Carbonate | 7,00% |
| Isopropyl Palmitate | 7,60% |
| Polysorbate 20 | 0,20% |
| Creatine | 0,50% |
| Panthenol | 0,50% |
| Glycerin | 3,00% |
| Water | ad 100% |
| TEGO® Carbomer 341 ER (Acrylates / C10-30 Alkyl Acrylate Cross-polymer) | 0,30% |
| Xanthan Gum | 0,10% |
| Sodium Hydroxide (10% in water) | q.s. |
| TEGO® Smooth Complex (Betaine; Urea; Potassium Lactate; Polygluta-mic Acid; Hydrolyzed Sclerotium Gum) | 2,00% |
| Preservative | q.s. |
| Perfume | q.s. |

Tabelle 7:

| Seidiges Cremegel: | |
|---|---|
| Beispiel | **5** |
| Emulgator 5 | 4,00% |
| Cyclomethicone | 10,00% |
| Dimethicone | 3,00% |
| Cetyl Ricinoleate | 2,00% |
| Xanthan Gum | 0,20% |
| TEGO® Carbomer 341 ER (Acrylates / C10-30 Alkyl Acrylate Cross-polymer) | 0,40% |
| Caprylic/Capric Triglyceride | 1,90% |
| Water | ad 100% |
| Alcohol | 5,00% |
| Sodium Hydroxide (10% in water) | q.s. |
| Preservative | q.s. |
| Perfume | q.s. |

Tabelle 8:

| O/W Tränkemulsion für kosmetische Feuchttücher: | | |
|---|---|---|
| | Beispiel | 6 |
| A | TEGO® Wipe DE (Diethylhexyl Carbonate; Polyglyceryl-4 Laurate; Phenoxyethanol; Methyl-paraben; Dilauryl Citrate; Ethylparaben; Butyl-paraben; Propylparaben; Isobutylparaben) | 5,70% |

(fortgesetzt)

| O/W Tränkemulsion für kosmetische Feuchttücher: | | |
|---|---|---|
| | Beispiel | 6 |
| B | Water, demineralized | 5,70% |
| C | Emulgator 1 | 0,30% |
| | Creatine | 0,25% |
| | Panthenol | 0,50% |
| | Water, demineralized | 87,55 |
| Z | Perfume | q.s. |

Herstellung: Bei Raumtemperatur wird zunächst A mit B gemischt, dann werden unter Rühren C und Z zugegeben.

<u>W/O Emulsionsbeispiele:</u>

Tabelle 9:

| Wasser-in-Siliconöl Lotion: | | | |
|---|---|---|---|
| Beispiel | **7** | **8** | **9** |
| Emulgator 3 | 2,00% | | |
| Emulgator 13 | | 2,00% | |
| Emulgator 17 | | | 2,00% |
| Cyclopentasiloxane | 19,50% | 19,50% | 19,50% |
| Sodium Chloride | 0,50% | 0,50% | 0,50% |
| Glycerin | 3,00% | 3,00% | 3,00% |
| Water | ad 100% | ad 100% | ad 100% |
| Preservative | q.s. | q.s. | q.s. |
| Perfume | q.s. | q.s. | q.s. |

Tabelle 10: Wasserfester Sonnenschutz:

| Beispiel | **10** | **11** | **12** |
|---|---|---|---|
| Emulgator 6 | 2,50% | | |
| Emulgator 15 | | 2,50% | |
| Emulgator 16 | | | 2,50% |
| C12-15 Alkyl Benzoate | 10,00% | 10,00% | 10,00% |
| Paraffinum Perliquidum | 13,50% | 13,50% | 13,50% |
| Cetyl Dimethicone | 1,00% | 1,00% | 1,00% |
| Titanium Dioxide | 5,00% | 5,00% | 5,00% |
| Sodium Chloride | 0,50% | 0,50% | 0,50% |
| Water | ad 100% | ad 100% | ad 100% |
| Preservative | q.s. | q.s. | q.s. |
| Perfume | q.s. | q.s. | q.s. |

Tabelle 11:

| Make-Up Grundierung (Make-Up Foundation): | | | |
|---|---|---|---|
| Beispiel | **13** | **14** | **15** |
| Emulgator 8 | 3,50% | | |
| Emulgator 15 | | 3,50% | |
| Emulgator 17 | | | 3,50% |
| Diethylhexyl Carbonate | 10,00% | 10,00% | 10,00% |
| Cyclopentasiloxane | 12,60% | 12,60% | 12,60% |
| Iron Oxides | 1,80% | 1,80% | 1,80% |
| Titanium Dioxide | 7,20% | 7,20% | 7,20% |
| Talc | 2,00% | 2,00% | 2,00% |
| Ethylhexyl Palmitate | 3,40% | 3,40% | 3,40% |
| Sodium Chloride | 1,00% | 1,00% | 1,00% |
| Glycerin | 2,00% | 2,00% | 2,00% |
| Water | ad 100% | ad 100% | ad 100% |
| Preservative | q.s. | q.s. | q.s. |
| Perfume | q.s. | q.s. | q.s. |

<u>Beispiele für die Verwendung als hydrophobes Emollient:</u>

Die folgenden zwei Beispiele sollen zeigen, dass erfindungsgemäße Produkte teilweise auch als hydrophobe bzw. lipophile Emollients in kosmetischen Formulierungen eingesetzt werden können und hier zu guten sensorischen Eigenschaften führen.

Tabelle 12:

| | Sonnenlotion W/O (in-vitro SPF 50+; Sun lotion): | | |
|---|---|---|---|
| | Beispiel | **16** | **17** |
| A | Isolan® GPS (Polyglyceryl-4 Diisostearate / Polyhydroxystearate / Sebacate) | 3,00% | 3,00% |
| | Produkt 10 | 0,40% | |
| | Produkt 11 | | 0,40% |
| | Zinc Stearate | 1,00% | 1,00% |
| | Tego® Sun TDEC 45 (Titanium Dioxide; Diethylhexyl Carbonate; Polyglyceryl-6 Polyhydroxystearate) | 22,00% | 22,00% |
| | Ethylhexyl Methoxycinnamate, Diethylamino Hydroxybenzoyl Hexyl Benzoate | 10,00% | 10,00% |
| | Ethylhexyl Salicylate | 5,00% | 5,00% |
| | Bis-Ethylhexyloxyphenol Methoxyphenyl Triazine (BEMT, Ciba) | 3,00% | 3,00% |
| | Preservative | q.s. | q.s. |
| B | Glycerin | 3,00% | 3,00% |
| | Magnesium Sulfate Heptahydrate | 1,50% | 1,50% |
| | Water | 51,10% | 51,10% |
| Z | Perfume | q.s. | q.s. |

Herstellung: A wird auf 80 °C aufgeheizt und unter Rühren B zugegeben und homogenisiert. Dann wird unter leichtem Rühren auf 30 °C abkühlen gelassen, Z hinzugegeben und nochmals homogenisiert.

Tabelle 13:

| O/W Handcreme (Hand Care Treatment): | | |
|---|---|---|
| | Beispiel | **18** |
| A | Glyceryl Stearate SE | 6,00% |
| | Cetearyl Alcohol | 2,00% |
| | Stearic Acid | 2,00 |
| | Ethylhexyl Stearate | 3,00% |
| | Mineral Oil (30 mPas) | 3,00% |
| | Dimethicone (350 mPas) | 1,50% |
| | Produkt 11 | 2,00% |
| B | Glycerin | 7,00% |
| | Panthenol | 0,50% |
| | Water | 73,00% |
| | Preservative, Perfume | q.s. |

Herstellung: A und B werden separat auf 70-75 °C aufgeheizt. Dann wird unter Rühren A zu B gegeben und homogenisiert. Unter leichtem Rühren wird abgekühlt.

Beispiele für Rinse-Off Anwendungen:

Austestung der Konditionierung von Haut (Hautpflegeleistung) und Schaumeigenschaften mittels eines Handwaschtests:

Zur Bewertung der Konditionierung von Haut (Hautpflegeleistung) und der Schaumeigenschaften des erfindungsgemäßen organomodifizierten, im Siliconteil verzweigten Siloxans Produkt Nr. 5 in wässrigen, tensidischen Formulierungen wurden sensorische Handwaschtests im Vergleich zu dem Vergleichsbeispiel Produkt Nr. 12 nach dem Stand der Technik durchgeführt.

Das Vergleichsprodukt 12 ist in der Industrie als Pflegewirkstoffe weit verbreitet und gilt als hochwirksamer Pflegewirkstoff in wässrigen, tensidischen Formulierungen. Eine Gruppe bestehend aus zehn trainierten Prüfpersonen wusch sich dabei definiert die Hände und bewertete Schaumeigenschaften und Hautgefühl anhand einer Notenskala von 1 (schlecht) bis 5 (sehr gut).
Die eingesetzten Produkte wurden jeweils in einer standardisierten Tensidformulierung (Tabelle 14) getestet. Als Kontrollformulierung 19 wird eine Formulierung ohne Zusatz eines Polyethersiloxans verwendet.

Tabelle 14:

| Testformulierungen für Handwaschtest: | | | |
|---|---|---|---|
| **Formulierungs-Beispiele** | **19** | **20** | **V21** |
| Texapon NSO®, 28%-ig, Cognis (INCI: Sodium Laureth Sulfate) | 32% | 32% | 32% |
| TEGO Betain F 50®, 38%-ig, Evonik Goldschmidt GmbH (INCI: Cocamidopropyl Betaine) | 8% | 8% | 8% |
| Sodium Chloride | 2% | 2% | 2% |
| Water, demineralized | ad 100,0% | | |
| Produkt Nr. 5 (erfindungsgemäß) | | 0,5% | |
| Produkt Nr. 12 (nicht erfindungsgemäß) | | | 0,5% |

Die sensorischen Testergebnisse sind in der Tabelle 15 zusammengefaßt.

Tabelle 15:

| Ergebnisse des Handwaschtests: | | | |
|---|---|---|---|
| **Testformulierung** | **19** | **20** | **V21** |
| Anschäumverhalten | 3,0 | 3,8 | 3,3 |
| Schaumvolumen | 2,8 | 3,4 | 3,0 |
| Schaumcremigkeit | 2,3 | 3,4 | 2,9 |
| Hautgefühl während des Waschens | 2,8 | 3,9 | 3,6 |
| Hautglätte | 1,4 | 3,0 | 2,7 |
| Hautweichheit | 2,0 | 2,9 | 2,6 |
| Hautglätte nach 3 min. | 2,6 | 3,8 | 3,4 |
| Hautweichheit nach 3 min. | 2,5 | 3,9 | 3,6 |

In Tabelle 15 sind die Ergebnisse des Handwaschtests dargestellt. Anhand der Meßergebnisse wird ersichtlich, dass die erfindungsgemäße Formulierung 20 unter Verwendung des erfindungsgemäßen Produkts Nr. 5 in allen Applikationseigenschaften im Vergleich zur Vergleichsformulierung V21 nach dem Stand der Technik überlegen ist. Vor diesem Hintergrund sind die Ergebnisse der erfindungsgemäßen Formulierung 20 als sehr gut zu bezeichnen. Anhand der Meßwerte ist ersichtlich, dass das erfindungsgemäße Produkt Nr. 5 in der Formulierung 20 zu einer Verbesserung der Hauteigenschaften und Schaumeigenschaften im Vergleich zu dem Produkt Nr. 12 in der Formulierung V21 führt. Ferner kann man den Meßwerten entnehmen, dass die Kontrollformulierung 19 ohne eine Polyethersiloxanverbindung schlechtere Meßwerte als die Formulierungen 20 und V21 aufweist.

Austestung der Konditionierung von Haar mittels Sensoriktests:

Für die anwendungstechnische Beurteilung der Konditionierung von Haar wurden das erfindungsgemäße Produkt Nr. 5 und das Vergleichsprodukt Nr. 12 in einfachen kosmetischen Formulierungen (Shampoo und Haarspülung) eingesetzt.

Die Anwendungseigenschaften beim Einsatz in einem Shampoo wurden in den folgenden Rezepturen überprüft:

Tabelle 16:

| Shampoo-Formulierungen zur Austestung der haarkonditionierenden Eigenschaften: | | | |
|---|---|---|---|
| **Formulierungs-Beispiele** | **22** | **23** | **V24** |
| Texapon NSO®, 28%-ig, Cognis (INCI: Sodium Laureth Sulfate) | 32% | 32% | 32% |
| TEGO® Betain F 50, 38%-ig, Evonik Goldschmidt GmbH (INCI: Cocamidopropyl Betaine) | 8% | 8% | 8% |
| Jaguar 162, Rhodia (INCI: Guar Hydroxypropyl trimonium Chloride) (Kationisches Polymer zur Verbesserung der Wirksamkeit von Konditioniermitteln) | 0,3% | 0,3% | 0,3% |
| Water, demineralized | ad 100,0% | | |
| Zitronensäure | ad. pH 6,0 $\pm$ 0,3 | | |
| Produkt Nr. 5 (erfindungsgemäß) | | 0,5% | |
| Produkt Nr. 12 (nicht erfindungsgemäß) | | | 0,5% |

Zur Bewertung der Eigenschaften der Shampooformulierung wurde im Testablauf keine Nachbehandlung mit einer Spülung durchgeführt.

Die Anwendungseigenschaften beim Einsatz in Haarspülungen wurden in den folgenden Rezepturen überprüft:

Tabelle 17:

| Haarspülung-Formulierungen zur Austestung der haarkonditionierenden Eigenschaften: | | | |
|---|---|---|---|
| **Formulierungs-Beispiele** | **25** | **26** | **V27** |
| TEGINACID®C, Evonik Goldschmidt GmbH (INCI: Ceteareth-25) | 0,5% | 0,5% | 0,5% |
| TEGO®Alkanol 16, Evonik Goldschmidt GmbH (INCI: Cetyl Alcohol)) | 4% | 4% | 4% |
| VARISOFT® 300, 30%-ig, Evonik Goldschmidt GmbH (INCI: Cetrimonium Chloride) | 3,3% | 3,3% | 3,3% |
| Water, demineralized | ad 100,0% | | |
| Zitronensäure | ad. pH 4,0 ± 0,3 | | |
| Produkt Nr. 5 (erfindungsgemäß) | | 0,5% | |
| Produkt Nr. 12 (nicht erfindungsgemäß) | | | 0,5% |

Die Vorbehandlung der Haare erfolgt im Falle der Eigenschaftsprüfung von Haarspülungen durch ein Shampoo, welches keine Konditioniermittel enthält.

Für die anwendungstechnische Beurteilung werden Haartressen, die für sensorische Tests verwendet werden, durch eine Dauerwellbehandlung und eine Bleichbehandlung standardisiert vorgeschädigt. Dazu werden friseurübliche Produkte verwendet. Der Testablauf, die verwendeten Basismaterialien sowie die Details der Beurteilungskriterien sind in DE 10327871 beschrieben.

Standardisierte Behandlung von vorgeschädigten Haarsträhnen mit konditionierenden Proben:

Die, wie oben beschrieben, vorgeschädigten Haarsträhnchen werden wie folgt mit dem oben beschriebenen Shampoo oder der oben beschriebenen konditionierenden Spülung behandelt:

Die Haarsträhnen werden unter fließendem, warmem Wasser benetzt. Das überschüssige Wasser wird leicht von Hand ausgedrückt, dann wird das Shampoo aufgebracht und sanft im Haar eingearbeitet (1 ml/Haarsträhne (2 g)). Nach einer Verweilzeit von 1 min wird das Haar für 1 min gespült. Gegebenenfalls wird direkt im Anschluss die Spülung aufgebracht und sanft im Haar eingearbeitet (1 ml/Haarsträhne (2 g) ). Nach einer Verweilzeit von 1 min wird das Haar für 1 min gespült.

Vor der sensorischen Beurteilung wird das Haar an der Luft bei 50% Luftfeuchtigkeit und 25 °C für mindestens 12 h getrocknet.

Beurteilungskriterien:

Die sensorischen Bewertungen erfolgen nach Noten, die auf einer Skala von 1 bis 5 vergeben werden, wobei 1 die schlechteste und 5 die beste Bewertung ist. Die einzelnen Testkriterien erhalten jeweils eine eigene Bewertung.

Die Testkriterien sind: Nasskämmbarkeit, Nassgriff, Trockenkämmbarkeit, Trockengriff, Aussehen/Glanz.

In der folgenden Tabelle 18 werden die Ergebnisse der sensorischen Beurteilung der wie oben beschrieben durchgeführten Behandlung der Haarsträhnen mit der erfindungsgemäßen Formulierung 23, der Vergleichsformulierung V24 und der Kontrollformulierung 22 (Placebo ohne Testsubstanz) verglichen.

Tabelle 18:

| Ergebnisse der Konditionierung von Haar aus Shampoo-Formulierung: | | | | | |
|---|---|---|---|---|---|
| | Nasskämmbarkeit | Nassgriff | Trockenkämmbarkeit | Trockengriff | Glanz |
| Erfindungsgemäße Formulierung 23 | 3,8 | 3,8 | 4,3 | 4,3 | 3,9 |
| Vergleichsformulierung V24 (nicht erfindungsgemäß) | 3,0 | 3,0 | 3,0 | 3,5 | 3,0 |

(fortgesetzt)

| Ergebnisse der Konditionierung von Haar aus Shampoo-Formulierung: | | | | | |
|---|---|---|---|---|---|
| | Nasskämmbarkeit | Nassgriff | Trockenkämmbarkeit | Trockengriff | Glanz |
| Kontrollformulierung 22 (Placebo) | 2,3 | 2,5 | 2,8 | 3,3 | 2,3 |

Die Ergebnisse zeigen in überraschender Weise, dass die erfindungsgemäße Formulierung 23 mit erfindungsgemäßem Produkt Nr. 5 signifikant bessere Bewertungen erhält, als die Vergleichsformulierung V24 mit dem Produkt Nr. 12 nach Stand der Technik. Besonders deutlich ist die gute Bewertung der Glanzeigenschaften aller erfindungsgemäßen Formulierungen hervorzuheben.

Tabelle 19:

| Ergebnisse der Konditionierung von Haar aus Haarspülungs-Formulierungen: | | | | | |
|---|---|---|---|---|---|
| | Nasskämmbarkeit | Nassgriff | Trockenkämmbarkeit | Trockengriff | Glanz |
| Erfindungsgemäße Formulierung 26 | 4,7 | 5,0 | 4,8 | 4,7 | 4,6 |
| Vergleichsformulierung V27(nicht erfindungsgemäß) | 4,5 | 4,2 | 4,5 | 4,3 | 3,8 |
| Kontrollformulierung 25 | 3,8 | 3,9 | 4,0 | 3,8 | 2,9 |

Auch in der Anwendung Haarspülung zeigt die erfindungsgemäße Formulierung 26 mit erfindungsgemäßem Produkt Nr. 5 in der sensorischen Beurteilung sehr gute kosmetische Bewertungen. Dabei wurden die bereits sehr guten Eigenschaften der Vergleichsformulierung V27 mit dem Vergleichsprodukt Nr. 12 durch die erfindungsgemäße Formulierung 26 mit der erfindungsgemäßen Verbindung Nr. 5 noch weiter gesteigert.
Eine signifikant bessere Bewertung wird auch beim Glanz durch die Verwendung der erfindungsgemäßen Formulierung 26 erreicht.
Weitere Formulierungsbeispiele:

Diese Beispiele sollen zeigen, dass die erfindungsgemäßen Siloxane in einer Vielzahl kosmetischer Formulierungen eingesetzt werden können.

Tabelle 20:

| Haarshampoo mit Konditionierer (Conditioning Shampoo): | |
|---|---|
| TEXAPON® NSO, Cognis, 28%-ig (INCI: Sodium Laureth Sulfate) | 25% |
| Perfume | 0,3% |
| Produkt Nr. 5 (erfindungsgemäß) | 0,5% |
| ABIL® Quat 3272, Evonik Goldschmidt GmbH (INCI: Quaternium-80) | 0,5% |
| PLANTACARE® 1200 UP, Cognis 50% (INCI: Lauryl Glycoside) | 4,25% |
| Water | 54,7% |
| TEGO® Betain F 50, Evonik Goldschmidt GmbH, 38%-ig (INCI: Cocamidopropyl Betaine) | 14,25% |
| ANTIL® 171, Evonik Goldschmidt GmbH (INCI: PEG-18 Glyceryl Oleate/Cocoate) | 0,5% |

Tabelle 21: Feuchtigkeitsspendende Körperwaschlotion (Moisturizing body wash):

| TEXAPON® NSO, Cognis, 28%-ig (INCI: Sodium Laureth Sulfate) | 30% |
|---|---|
| TEGOSOFT® PC 31, Evonik Goldschmidt GmbH (INCI: Polyglyceryl-3 Caprate) | 0,5% |

(fortgesetzt)

| | |
|---|---|
| Produkt Nr. 5 (erfindungsgemäß) | 0,3% |
| Perfume | 0,3% |
| Water | 54,1% |
| TEGOCEL® HPM 4000, Evonik Goldschmidt GmbH (INCI: Hydroxypropyl Methylcellulose) | 0,3% |
| REWOTERIC® AM C, Evonik Goldschmidt GmbH, 32%-ig (INCI: Sodium Cocoamphoacetate) | 10% |
| Citric Acid Monohydrate | 0,5% |
| REWODERM® LI S 80, Evonik Goldschmidt GmbH (INCI: PEG-200 Hydrogenated Glyceryl Palmate; PEG-7 Glyceryl Cocoate) | 2% |
| TEGO® Pearl N 300, Evonik Goldschmidt GmbH (INCI: Glycol Distearate; Laureth-4; Cocamidopropyl Betaine) | 2% |

Tabelle 22: Körperreinigungsschaum (Body cleansing Foam):

| | |
|---|---|
| TEXAPON® NSO, Cognis, 28%-ig (INCI: Sodium Laureth Sulfate) | 14% |
| Perfume | 0,3% |
| Produkt Nr. 5 (erfindungsgemäß) | 0,5% |
| REWOTERIC® AM C, Evonik Goldschmidt GmbH, 32%-ig (INCI: Sodium Cocoamphoacetate) | 8% |
| Water | 75,2% |
| TEGOCEL® HPM 50, Evonik Goldschmidt GmbH (INCI: Hydroxypropyl Methylcellulose) | 0,5% |
| LACTIL®, Evonik Goldschmidt GmbH (INCI: Sodium Lactate; Sodium PCA; Glycine; Fructose; Urea; Niacinamide; Inositol; Sodium benzoate; Lactic Acid) | 1% |
| Citric Acid Monohydrate | 0,5% |

Tabelle 23:Mildes Duschbad (Mild shower Bath):

| | |
|---|---|
| TEXAPON® NSO, Cognis, 28%-ig (INCI: Sodium Laureth Sulfate) | 27% |
| REWOPOL® SB FA 30, Evonik Goldschmidt GmbH, 40%-ig (INCI: Disodium Laureth Sulfosuccinate) | 12% |
| TEGOSOFT® LSE 65 K SOFT, Evonik Goldschmidt GmbH (INCI: Sucrose Cocoate) | 2% |
| Water | 39% |
| REWOTERIC® AM C, Evonik Goldschmidt GmbH, 32%-ig (INCI: Sodium Cocoamphoacetate) | 13% |
| Produkt Nr. 5 (erfindungsgemäß) | 0,5% |
| Citric Acid (30% in water) | 3% |
| ANTIL® 171 Evonik Goldschmidt GmbH (INCI: PEG-18 Glyceryl Oleate/Cocoate) | 1,5% |
| TEGO® Pearl N 300 Evonik Goldschmidt GmbH (INCI: Glycol Distearate; Laureth-4; Cocamidopropyl Betaine) | 2% |

Tabelle 24: Haar-Konditionierer (Hair Conditioner):

| A | Water | 93,75% |
|---|---|---|
| | Propylene Glycol | 1% |
| | Citric acid Monohydrate | q.s. |
| B | TEGO® Alkanol 16, Evonik Goldschmidt GmbH (INCI: Cetyl alcohol) | 3% |
| | VARISOFT® PATC, Evonik Goldschmidt GmbH (INCI: Palmitamidopropyltrimonium Chloride) | 1,75% |
| | Produkt Nr. 5 (erfindungsgemäß) | 0,5% |
| C | Perfume, preservative | q.s. |

Tabelle 25: Haar-Reparatur-Spray und Konditionierer (Hair repair leave-in Conditioner spray):

| TAGAT® CH-40, Evonik Goldschmidt GmbH (INCI: PEG-40 Hydrogenated Castor Oil) | 2% |
|---|---|
| Ceramide VI, Evonik Goldschmidt GmbH (INCI: Ceramide 6 II) | 0,05% |
| Perfume | 0,2% |
| Water | 89,75% |
| Produkt Nr. 5 (erfindungsgemäß) | 2,0% |
| LACTIL® Evonik Goldschmidt GmbH (INCI: Sodium Lactate; Sodium PCA; Glycine; Fructose; Urea; Niacinamide; Inositol; Sodium benzoate; Lactic Acid) | 2% |
| TEGO® Betain F 50 Evonik Goldschmidt GmbH 38% (INCI: Cocamidopropyl Betaine) | 2% |
| Citric Acid (10% in water) | 2% |

Tabelle 26: Konditionier-Schaum (Leave-in Conditioning Mousse):

| Produkt Nr. 5 (erfindungsgemäß) | 0,5% |
|---|---|
| ABIL® B 88183, Evonik Goldschmidt GmbH (INCI: PEG/PPG-20/6 Dimethicone) | 0,4% |
| TAGAT® CH-40 (INCI: PEG-40 Hydrogenated Castor Oil) | 0,5% |
| Perfume | 0,2% |
| TEGO® Betain 810, Evonik Goldschmidt GmbH, 38%-ig (INCI: Capryl/Capramidopropyl Betaine) | 4% |
| Water | 93,5% |
| Panthenol | 0,2% |
| LACTIL®, Evonik Goldschmidt GmbH (INCI: Sodium Lactate; Sodium PCA; Glycine; Fructose; Urea; Niacinamide; Inositol; Sodium benzoate; Lactic Acid) | 0,3% |
| Citric Acid (30% in water) | 0,4% |

Tabelle 27: Cremiger Rasierschaum (Creamy Shaving Foam):

| A | Water | 50% |
|---|---|---|
| | Coconut Fatty Acid | 1,4% |
| | Monoethanolamine | 1,3% |
| | Myristic Acid | 3,5% |
| B | TEGOSOFT® LSE 65 K Evonik Goldschmidt (INCI: Sucrose Cocoate) | 2% |

(fortgesetzt)

| C | TEGO® Betain 810 Evonik Goldschmidt (INCI: Capryl/Capramidopropyl Betaine) | 7,6% |
|---|---|---|
| | Glycerin | 5% |
| | Produkt Nr. 5 (erfindungsgemäß) | 1,7% |
| | Perfume | 0,3% |
| | Water | 26,5% |
| | TEGOCEL® HPM 50 Evonik Goldschmidt (INCI: Hydroxypropyl Methylcellulose) | 0,7% |

**Patentansprüche**

1. Verwendung von organomodifizierten, im Siloxanteil verzweigten Siloxanen als Additive, Emulgatoren, Dispergierhilfsmittel und/oder Pflegewirkstoffe in kosmetischen, dermatologischen und pharmazeutischen Formulierungen **dadurch gekennzeichnet,**
**dass** die Siloxane als Additive für kosmetische und pharmazeutische Formulierungen zur Verbesserung der sensorischen Eigenschaften und/oder zur Konditionierung von Haut und/oder Haar eingesetzt werden, und dass Siloxane der Formel I

$$M_a\, M'_b\, D_c\, D'_d\, T_e\, Q_f \qquad \text{Formel I}$$

wobei

$M = (R^1_3\, Si\, O_{1/2})$
$M' = (R^2 R^1_2\, Si\, O_{1/2})$
$D = (R^1_2\, Si\, O_{2/2})$
$D' = (R^2 R^1\, Si\, O_{2/2})$
$T = (R^3\, Si\, O_{3/2})$
$Q = (Si\, O_{4/2})$
$a = 0$ bis $14$;
$b = 1$ bis $14$;

mit der Maßgabe, dass

$$a + b > 3;$$

$c = 20$ bis $400$;
$d = 0$;
$e = 0$ bis $15$;
$f = 0$ bis $10$;

mit der Maßgabe, dass

$$e + f \geq 3;$$

$R^1 = $ Methyl,
$R^2 = $ unabhängig voneinander gleiche oder verschiedene lineare oder verzweigte Alkylreste mit mehr als 7 Kohlenstoffatomen oder, Polyetherreste der allgemeinen Formel IIa $-CH_2-CH_2-(CH_2)_n O(EO)_x (PO)_y (XO)_z R^4$ Formel IIa,

mit

$EO = (C_2 H_4 O)$;

PO = $(C_3H_6O)$;

XO = $(C_2H_3R^5O)$ ;

n = 0 bis 9;

x = 5 bis 30;

y = 0 bis 20;

z = 0;

$R^4$ = H,

$R^5$ = unabhängig voneinander gleiche oder verschiedene Reste ausgewählt aus der Gruppe umfassend: Alkylreste mit 2 bis 16 C-Atomen, die durch Etherfunktionen unterbrochen sein können, Alkarylreste mit 7 bis 18 C-Atomen, Arylreste mit 6 bis 16 C-Atomen; entspricht;

$R^3$ = unabhängig voneinander gleiche oder verschiedene Reste $R^1$ oder $R^2$,

verwendet werden.

2. Verwendung nach Anspruch 1, **dadurch gekennzeichnet, dass** die Siloxane hergestellt werden durch Hydrosilylierung von ungesättigten Polyetherderivaten mit im Siliconteil verzweigten, bei einer Temperatur von 25 °C und einem Druck von 101325 Pa flüssigen SiH-funktionellen Siloxanblockcopolymeren, wobei die SiH-funktionellen Siloxanblockcopolymere hergestellt wurden, indem eine Mischung, enthaltend

a) ein oder mehrere SiH-funktionelle Siloxane,

b) ein oder mehrere SiH-Funktion-freie Siloxane und

c) ein oder mehrere Tetraalkoxysilane,

und gegebenenfalls

d) ein oder mehrere Trialkoxysilane unter Zugabe von Wasser und in Anwesenheit von mindestens einem festen Brönstedt-sauren Katalysator, welcher ausgewählt ist aus den sauren Ionenaustauschern, in einem Verfahrensschritt umgesetzt werden.

3. Verwendung nach zumindest einem der Ansprüche 1 oder 2, **dadurch gekennzeichnet, dass** die Siloxane als Emulgatoren in Öl-in-Wasser- (O/W) und/oder Wasser-in-Öl- (W/O) Emulsionen eingesetzt werden.

4. Verwendung nach zumindest einem der Ansprüche 1 bis 3, **dadurch gekennzeichnet, dass** die Formulierungen anorganische oder organische Pigmente und/oder anorganische oder organische Partikel enthalten.

5. Verwendung nach einem oder mehreren der vorstehenden Ansprüche als Emulgatoren in Pflegecremes und -lotionen für Gesicht, Körper und Hände, in Sonnenschutzemulsionen, in Schminken, in Aersolen, Roll-ons, Pumpsprays, Stiften, im AP/Deo-Bereich, in Babypflegeprodukten, in Intimpflege-, Fußpflege-, Haarpflege-, Nagelpflege-, Zahnpflege- oder Mundpflegeprodukten sowie in dermatologischen Salben.

6. Verwendung nach einem oder mehreren der vorstehenden Ansprüche in wässrigen Pflegeformulierungen oder tensidischen kosmetischen, dermatologischen oder pharmazeutischen Formulierungen für Duschbäder und/oder -gele, Badeformulierungen, Flüssigseifen und Shampoos, Hautmasken, Rasierschäume, Haarspülungen, Leave-in Conditioner und/oder Stylingprodukte für Haar.

7. Zubereitungen enthaltend organomodifizierte, im Siliconteil verzweigte Siloxane der Formel I nach Anspruch 1.

8. Kosmetische, dermatologische oder pharmazeutische Formulierung enthaltend mindestens eine der Zubereitungen gemäß Anspruch 7 in Form einer Emulsion.

9. Kosmetische, dermatologische oder pharmazeutische Formulierung gemäß Anspruch 8 als zusätzliche Komponente enthaltend Partikel oder Pigmente.

10. Kosmetische, dermatologische oder pharmazeutische Formulierung gemäß Anspruch 8 als zusätzliche Komponente enthaltend kosmetische Wirkstoffe.

**Claims**

1. Use of organomodified siloxanes branched in the siloxane part as additives, emulsifiers, dispersion auxiliaries and/or care active ingredients in cosmetic, dermatological and pharmaceutical formulations, **characterized in that** the siloxanes are used as additives for cosmetic and pharmaceutical formulations for improving sensory properties and/or for the conditioning of skin and/or hair, and **in that** siloxanes of the formula I

$$M_a M'_b D_c D'_d T_e Q_f \qquad \text{formula I}$$

wherein

$M = (R^1_3 SiO_{1/2})$
$M' = (R^2 R^1_2 SiO_{1/2})$
$D = (R^1_2 SiO_{2/2})$
$D' = (R^2 R^1 SiO_{2/2})$
$T = (R^3 SiO_{3/2})$
$Q = (SiO_{4/2})$
$a$ = 0 to 14;
$b$ = 1 to 14;

with the proviso that

$$a + b > 3;$$

$c$ = 20 to 400;
$d$ = 0;
$e$ = 0 to 15;
$f$ = 0 to 10;

with the proviso that

$$e + f \geq 3;$$

$R^1$ = methyl,
$R^2$ = independently of one another, identical or different linear or branched alkyl radicals having more than 7 carbon atoms polyether radicals of the general formula IIa

$$-CH_2-CH_2-(CH_2)_n O(EO)_x(PO)_y(XO)_z R^4 \qquad \text{formula IIa,}$$

where

$EO = (C_2H_4O)$ ;
$PO = (C_3H_6O)$;
$XO = (C_2H_3R^5O)$ ;
$n$ = 0 to 9;
$x$ = 5 to 30;
$y$ = 0 to 20;
$z$ = 0;
$R^4$ = H,
$R^5$ = independently of one another, identical or different radicals selected from the group comprising: alkyl radicals having 2 to 16 carbon atoms, which may be interrupted by ether functions, alkaryl radicals having 7 to 18 carbon atoms, aryl radicals having 6 to 16 carbon atoms;
$R^3$ = independently of one another, identical or different radicals $R^1$ or $R^2$,

are used.

**2.** Use according to Claim 1, **characterized in that** the siloxanes are prepared by hydrosilylation of unsaturated polyether derivatives with SiH-functional siloxane block copolymers branched in the silicone part and liquid at a temperature of 25°C and a pressure of 101 325 Pa, where the SiH-functional siloxane block copolymers were prepared by reacting a mixture comprising

    a) one or more SiH-functional siloxanes,
    b) one or more SiH-function-free siloxanes and
    c) one or more tetraalkoxysilanes,

and optionally

    d) one or more trialkoxysilanes
    with the addition of water and in the presence of at least one solid Brönsted-acidic catalyst, which is selected from the acidic ion exchangers, in one process step.

**3.** Use according to at least one of Claims 1 and 2, **characterized in that** the siloxanes are used as emulsifiers in oil-in-water (O/W) and/or water-in-oil (W/O) emulsions.

**4.** Use according to at least one of Claims 1 to 3, **characterized in that** the formulations comprise inorganic or organic pigments and/or inorganic or organic particles.

**5.** Use according to one or more of the preceding claims as emulsifiers in care creams and lotions for face, body and hands, in sunscreen emulsions, in make-up, in aerosols, roll-ons, pump sprays, sticks, in the antiperspirant/deodorant sector, in babycare products, in intimate care, footcare, haircare, nail care, dental care or oral care products, and also in dermatological ointments.

**6.** Use according to one or more of the preceding claims in aqueous care formulations or surface-active, cosmetic, dermatological or pharmaceutical formulations for shower baths and/or shower gels, bath formulations, liquid soaps and shampoos, skin masks, shaving foams, hair rinses, leave-in conditioners and/or styling products for hair.

**7.** Preparations comprising organomodified siloxanes of formula I branched in the silicone part according to Claim 1.

**8.** Cosmetic, dermatological or pharmaceutical formulation comprising at least one of the preparations according to Claim 7 in the form of an emulsion.

**9.** Cosmetic, dermatological or pharmaceutical formulation according to Claim 8 comprising particles or pigments as additional component.

**10.** Cosmetic, dermatological or pharmaceutical formulation according to Claim 8 comprising cosmetic active ingredients as additional component.

**Revendications**

**1.** Utilisation de siloxanes organomodifiés, ramifiés dans la partie siloxane, comme additifs, émulsifiants, adjuvants de dispersion et/ou substances actives de soin dans des formulations cosmétiques, dermatologiques et pharmaceutiques, **caractérisée en ce que** les siloxanes sont utilisés comme additifs pour des formulations cosmétiques et pharmaceutiques pour l'amélioration des propriétés sensorielles et/ou pour le conditionnement de la peau et/ou des cheveux et **en ce que** des siloxanes de formule I

$$M_a M'_b D_c D'_d T_e Q_f \qquad \text{Formule I}$$

dans laquelle

M = $(R^1_3 SiO_{1/2})$
M' = $(R^2 R^1_2 SiO_{1/2})$
D = $(R^1_2 SiO_{2/2})$
D' = $(R^2 R^1 SiO_{2/2})$

$T = (R^3SiO_{3/2})$
$Q = (SiO_{4/2})$
$a = 0$ à 14 ;
$b = 1$ à 14 ;

étant entendu que

$$a + b > 3 ;$$

$c = 20$ à 400 ;
$d = 0$ ;
$e = 0$ à 15 ;
$f = 0$ à 10 ;

étant entendu que

$$e + f \geq 3 ;$$

$R^1$ = méthyle,
$R^2$ = indépendamment, des radicaux alkyle identiques ou différents, linéaires ou ramifiés, comprenant plus de 7 atomes de carbone ou des radicaux polyéther présentant la formule générale IIa $-CH_2-CH_2-(CH_2)_nO(EO)_x(PO)_y(XO)_zR^4$ Formule IIa,

dans laquelle

$EO = (C_2H_4O)$ ;
$PO = (C_3H_6O)$ ;
$XO = (C_2H_3R^5O)$ ;
$n = 0$ à 9 ;
$x = 5$ à 30 ;
$y = 0$ à 20 ;
$z = 0$ ;
$R^4 = H$,
$R^5$ = indépendamment, des radicaux identiques ou différents choisis dans le groupe comprenant : les radicaux alkyle comprenant 2 à 16 atomes de carbone qui peuvent être interrompus par des fonctions éther, des radicaux alkaryle comprenant 7 à 18 atomes de carbone, des radicaux aryle comprenant 6 à 16 atomes de carbone ;
$R^3$ = indépendamment, des radicaux $R^1$ ou $R^2$ identiques ou différents

sont utilisés.

2. Utilisation selon la revendication 1, **caractérisée en ce que** les siloxanes sont préparés par hydrosilylation de dérivés insaturés de polyéthers avec des copolymères séquencés de siloxane à fonctionnalité SiH, ramifiés dans la partie silicone, liquides à une température de 25°C et à une pression de 101325 Pa, les copolymères séquencés de siloxane à fonctionnalité SiH ayant été préparés par transformation d'un mélange, contenant

   a) un ou plusieurs siloxanes à fonctionnalité SiH,
   b) un ou plusieurs siloxanes exempts de fonction SiH et
   c) un ou plusieurs tétraalcoxysilanes et le cas échéant
   d) un ou plusieurs trialcoxysilanes
   avec addition d'eau et en présence d'au moins un catalyseur solide, acide de Brönstedt, qui est choisi parmi les échangeurs d'ions acides, dans une étape de procédé.

3. Utilisation selon au moins l'une quelconque des revendications 1 ou 2, **caractérisée en ce que** les siloxanes sont utilisés comme émulsifiants dans des émulsions huile-dans-eau (H/E) et/ou des émulsions eau-dans-huile (E/H).

**4.** Utilisation selon au moins l'une quelconque des revendications 1 à 3, **caractérisée en ce que** les formulations contiennent des pigments inorganiques ou organiques et/ou des particules inorganiques ou organiques.

**5.** Utilisation selon l'une ou plusieurs des revendications précédentes comme émulsifiants dans des crèmes et des lotions de soin pour le visage, le corps et les mains, dans des émulsions de protection solaire, dans des maquillages, dans des aérosols, des systèmes d'application à bille, des sprays à pompe, des bâtons, dans le domaine antiperspirant/déodorant, dans des produits de soin pour les bébés, dans des produits d'hygiène intime, de soin des pieds, des cheveux, des ongles, des dents ou de la bouche ainsi que dans des pommades dermatologiques.

**6.** Utilisation selon l'une ou plusieurs des revendications précédentes dans des formulations aqueuses de soin ou dans des formulations tensioactives cosmétiques, dermatologiques ou pharmaceutiques pour des bains-douches et/ou des gels-douches, des formulations pour le bain, des savons liquides et des shampooings, des masques pour la peau, des mousses de rasage, des après-shampooings, des revitalisants sans rinçage et/ou des produits de coiffage pour les cheveux.

**7.** Préparations contenant des siloxanes organomodifiés, ramifiés dans la partie silicone de formule I selon la revendication 1.

**8.** Formulation cosmétique, dermatologique ou pharmaceutique contenant au moins une des préparations selon la revendication 7 sous forme d'une émulsion.

**9.** Formulation cosmétique, dermatologique ou pharmaceutique selon la revendication 8 comme composant supplémentaire contenant des particules ou des pigments.

**10.** Formulation cosmétique, dermatologique ou pharmaceutique selon la revendication 8 comme composant supplémentaire contenant des substances actives cosmétiques.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 1125574 A
- EP 0298402 A
- EP 1416016 A
- US 5474712 A
- WO 02053111 A
- US 5879671 A
- DE 19603357

- DE 102008041601
- DE 102007055485
- EP 1520870 A
- DE 102008001788
- DE 10327871

**In der Beschreibung aufgeführte Nicht-Patentliteratur**

- **DR. KOLLMEIER.** Les Copolymeres Polysiloxanes polyethers comme additifs dans les formulations cosmetiques. *Parfums, cosmetiques, aromes,* 1983, vol. 51, 67-72